Europäisches Patentamt

European Patent Office

Office européen des brevets

(11) Numéro de publication: **0 406 136 A1**

## (12) DEMANDE DE BREVET EUROPEEN

(21) Numéro de dépôt: 90420306.4

(22) Date de dépôt: 27.06.90

(51) Int. Cl.⁵: **B65B 61/18, B65D 75/60**

(30) Priorité: 29.06.89 FR 8909067

(43) Date de publication de la demande:
02.01.91 Bulletin 91/01

(84) Etats contractants désignés:
AT BE CH DE DK ES GB GR IT LI LU NL SE

(71) Demandeur: **Marion, Louis**
**Vieux Bourg de Condamine**
**F-42230 Roche la Moliére(FR)**

(72) Inventeur: **Marion, Louis**
**Vieux Bourg de Condamine**
**F-42230 Roche la Moliére(FR)**

(74) Mandataire: **Dupuis, François**
**Cabinet Laurent et Charras, 3 Place de**
**l'Hôtel-de-Ville, BP 203**
**F-42005 St. Etienne Cédex 1(FR)**

(54) **Procédé et moyens de fabrication de sachets en papier pour emballer des produits notamment des produits stérilisés, et les sachets obtenus.**

(57) Procédé et moyens de fabrication de sachets en papier pour emballer des produits notamment des produits stérilisés, et les sachets obtenus.

Le procédé est remarquable en ce qu'on exécute au moins une ligne (L) d'affaiblissement du papier sur au moins une des deux bandes (B1 - B2) consitutant le sachet (S) ; l'épaisseur de papier suivant cette ou ces lignes étant calculée pour que le sachet conserve toute son étanchéité et une bonne résistance à la traction de part et d'autre de la ou des lignes d'affaiblissement, et pour être facilement ouvert par une action manuelle de cisaillement au niveau de la ou desdites lignes.

FIG.7

## PROCÉDÉ ET MOYENS DE FABRICATION DE SACHETS EN PAPIER POUR EMBALLER DES PRODUITS NOTAMMENT DES PRODUITS STÉRILISÉS, ET LES SACHETS OBTENUS.

L'objet de l'invention se rattache aux secteurs techniques de la manipulation des matériaux en bandes, ainsi que des conditionnements et emballage en général.

Un certain nombre de produits généralement en matière souple et plus particulièrement en matériau textile tissé ou non tissé, doivent être conditionnés dans des sachets en papier. C'est le cas notamment pour les compresses chirurgicales qui sont emballées unitairement ou par groupage de x exemplaires et de manière étanche et stérile, entre deux feuilles ou bandes de papier spécial réunies entre elles par collage selon tout procédé et moyens automatisés bien connus.

Le papier utilisé pour conditionner ce type de produit est soit du papier totalement enduit sur les faces en contact des deux bandes pour être collés l'un à l'autre tout autour de la ou des compresses, soit un papier préencollé en bordure appelé "papier pelable", selon lequel lorsqu'on ouvre le sachet, la colle se retrouve d'un seul côté afin d'éviter une réutilisation du sachet.

La fermeture du sachet est généralement réalisée par scellage à chaud ou à froid à l'aide de molettes, rouleaux de pression ou organes similaires, agissant longitudinalement et transversalement de part et d'autre des bandes de papier superposées entre lesquelles ont été préalablement déposées le compresses, puis un outil de coupe transversal assure la séparation périodique des bandes superposées et collées, entre les empilements de compresses. Tout cela est bien entendu réalisé en continu et de manière automatique par des machines ou dispositifs appropriés. Par exemple et non limitativement, avec l'installation ayant fait l'objet de la Demande de Brevet en France N° 88. 16490 dont le déposant est également le titulaire.

Si ce type d'emballage donne satisfaction quant à l'étanchéité et à la solidité, il présente par contre un certain nombre d'inconvénients lorsqu'il s'agit d'ouvrir le sachet ainsi réalisé. En effet, pour avoir accès au contenu du sachet, il faut déchirer manuellement à partir d'un des bords ou bien couper avec des ciseaux, les deux épaisseurs de papier. Ces opérations ne sont pas toujours réussies d'un seul coup. Par exemple, la déchirure ou la coupure n'a pas été réalisée correctement en dedans de la soudure périphérique, et il faut recommencer. On risque également de détériorer une ou plusieurs compresses si le coup de ciseaux ou la déchirure a été trop importante. On peut encore ouvrir le sachet en séparant à partir d'un angle ou d'un point indiqué les deux épaisseurs de papier et en tirant suivant des directions inverses, mais cela n'est pas très aisé à réaliser même lorsqu'il est prévu une encore d'amorce de l'ouverture. De plus, il peut arriver, lors de ces actions plus ou moins désordonnées, que le contenu du sachet s'échappe inopinément, et on comprend que dans ce cas toute stérilisation préalable devient caduque. A noter également que l'on produit souvent de petits déchets bien encombrants ou s'infiltrant partout.

C'est donc pour supprimer ces inconvénients que l'on a conçu les sachets selon l'invention qui permettent par une action manuelle, simple et rapide de libérer le contenu du sachet sans risque de le détériorer et tout en conservant les qualités de solidité, d'inviolabilité et de propreté de l'emballage.

Pour cela, et selon une première caractéristique, on exécute, au moins une ligne d'affaiblissement du papier sur au moins une des deux bandes constituant le sachet ; l'épaisseur de papier suivant cette ou ces lignes étant calculée pour que le sachet conserve toute son étanchéité et une bonne résistance à la traction de part et d'autre de la ou des lignes d'affaiblissement, et pour être facilement ouvert par une action manuelle de cisaillement au niveau de la ou desdites lignes.

Suivant d'autres caractéristiques, la ou les lignes d'affaiblissement sont réalisées par passage de la ou des bandes de papier constituant le sachet entre une ou des molettes de pression et un ou des galets de contre-appui, disposés sur le parcours de la ou des bandes soit entre la ou les bobines de papier et le poste d'introduction du ou des produits entre les deux bandes de papier superposées, soit avant constitution de la ou des bobines.

Suivant d'autres caractéristiques, les sachets réalisés avec du papier pelable présentent au moins une ligne d'affaiblissement exécutée sur seule des deux bandes le constituant, tandis que les sachets réalisés avec du papier totalement enduit présentent au moins une ligne d'affaiblissement exécutée sur chacune des bandes le constituant, la ou lesdites lignes étant alignées en superposition.

Ces caractéristiques et d'autres encore ressortiront de la description qui suit.

Pour fixer l'objet de l'invention, sans toutefois le limiter, dans les dessins annexés :

La figure 1 est une vue très schématique illustrant une installation d'ensachage de compresses équipée des moyens de réalisation des lignes d'affaiblissement.

Les figures 2, 3 et 4 sont des vues en perspective illustrant plusieurs formes de réalisation des lignes d'affaiblissement.

La figure 5 est une vue en coupe transversale à plus grande échelle illustrant un sachet avec des lignes d'affaiblissement.

La figure 6 est une vue de dessus illustrant la résistance à la traction manuelle d'un sachet réalisé avec une ligne d'affaiblissement.

Les figures 7 et 8 sont des vues en perspective illustrant l'opération d'ouverture d'un sachet à une ou plusieurs lignes d'affaiblissement.

Afin de rendre plus concret l'objet de l'invention, on le décrit maintenant d'une manière non limitative en se référant aux exemples de réalisation des figures des dessins.

Comme on l'a dit plus haut, la réalisation de la ou des lignes d'affaiblissement sur l'une des bandes constituant le sachet ou les deux peut s'opérer en tout point d'une installation d'ensachage existante, entre le poste de distribution des bandes et le poste d'introduction du ou des compresses entre les deux bandes mises en superposition. Il n'est pas exclu cependant de prévoir la réalisation de la ou des lignes d'affaiblissement avant la mise en bobine du papier destiné à former le sachet.

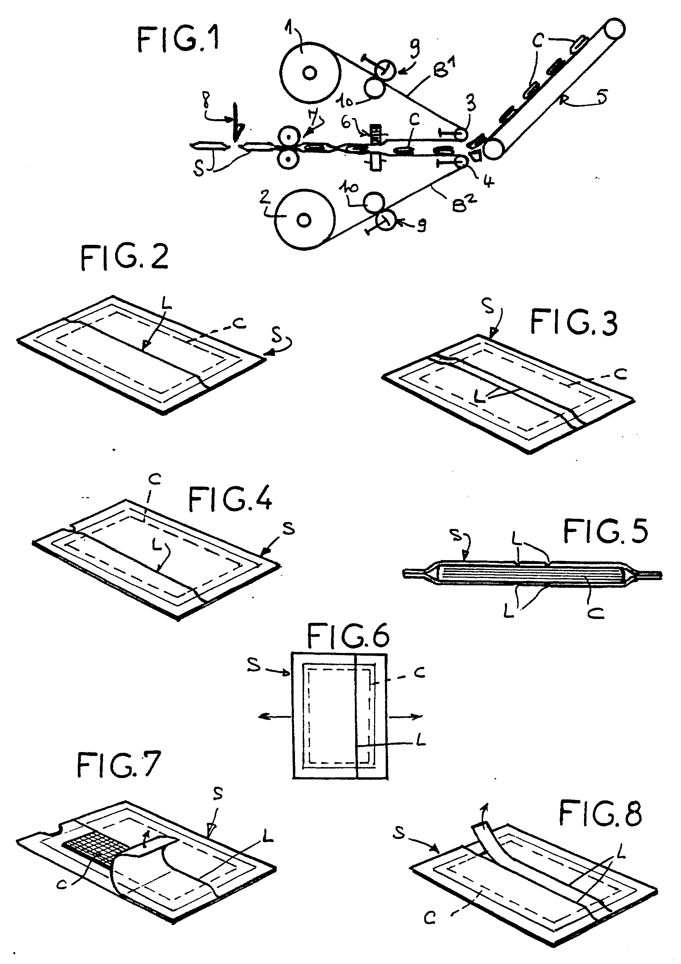

On a illustré schématiquement à la figure 1 un exemple d'installation d'ensachage intégrant les moyens de réalisation des lignes d'affaiblissement.

On voit des bobines (1) et (2) de papier enduit ou préencollé, des rouleaux de renvoi (3) et (4) situés à la base d'un tapis (5) d'amenée des compresses (C) qui doivent être introduites entre les deux bandes de papier (B1 - B2), des moyens (6) et (7) complémentaires pour assurer par pression la soudure transversale et longitudinale des bandes afin de constituer des chapelets de sachets (S) qui sont ensuite détachés par un organe de coupe transversale (8).

Entre les bobines (1) et (2) et les rouleaux de renvoi (3) et (4), les bandes de papier passent entre une molette (9) et un galet de contre-appui (10) qui sont agencés pour réaliser une empreinte dans les bandes par écrasement et donc un affaiblissement à cet endroit. L'un des éléments, de préférence la molette (9) est montée avec une capacité de réglage en position pour ajuster très précisément la profondeur de marquage en fonction du type de papier et de son épaisseur, tout en gardant à l'esprit que le sachet confectionné doit conserver toute son étanchéité et une bonne résistance aux tractions manuelles perpendiculairement opposées à la ligne d'affaiblissement (L) réalisés, comme le montre la figure 6.

Suivant les besoins et suivant que le papier d'emballage est préencollé ou totalement enduit, on exécute la ou les lignes d'affaiblissement de différentes manières.

Le sachet illustré à la figure 2 est par exemple du type pelable. Dans ce cas, une seule ligne d'affaiblissement (L) sur une des bandes est suffisante pour ouvrir aisément le sachet en agissant manuellement à partir d'une des extrémités de la ligne, comme on le voit à la figure 7. Bien entendu, on peut également prévoir deux lignes (L) espacées sur l'une des bandes, comme illustré à la figure 8, afin de détacher, toujours par cisaillement, une mince bande de papier, ce qui permet de tenir la compresse après détachement de ladite bande.

Comme montré à la figure 4, si l'une des bandes de papier pelable présente une encoche (E) destinée à faciliter l'ouverture du sachet, on prévoit alors d'exécuter la ligne d'affaiblissement (L) à partir de cette encoche.

Dans le cas de sachet en papier totalement enduit, il est nécessaire de prévoir une ou deux lignes d'affaiblissement (L) sur chacune des bandes (B1 - B2), comme illustré aux figures 3, 5 et 8 ; la ou lesdites lignes de chaque bande étant en alignement.

Il est évident que la ou les lignes d'affaiblissement peuvent être réalisées aussi bien transversalement que longitudinalement, et soit en bordure, soit dans la zône centrale des sachets.

Les avantages ressortent bien de la description ; on souligne notamment :
- la facilité d'ouverture du sachet sans création de déchets de papier et sans détérioration du contenu ;
= l'inviolabilité du contenu du sachet et sa résistance aux manipulations, garantissant la stérilisation ;
- la mise en oeuvre aisée des moyens de marquage des lignes sur toute installation existante ;
- la sûreté d'utilisation, en particulier la bonne tenue en main des produits délicats.

## Revendications

-1- Procédé de fabrication de sachets en papier pour emballer des produits, notamment des produits stérilisés, à partir de deux bandes de papier amenées en superposition et entre lesquelles sont introduits les produits à emballer qui y sont enfermés de manière étanche par scellage périphérique desdites bandes entre elles ; le procédé est caractérisé en ce qu'on exécute au moins une ligne (L) d'affaiblissement du papier sur au moins une des deux bandes (B1 - B2) constituant le sachet (S) ; l'épaisseur de papier suivant cette ou ces lignes étant calculée pour que le sachet conserve toute son étanchéité et une bonne résistance à la traction de part et d'autre de la ou des lignes d'affaiblissement, et pour être facilement ouvert par une action manuelle de cisaillement au niveau de la ou desdites lignes.

-2- Procédé selon la revendication 1, caractérisé en ce qu'on exécute la ou les lignes (L) d'affaiblissement du papier sur l'une des bandes ou sur les deux bandes destinées à constituer le sachet, avant la mise en bobine desdites bandes.

-3- Procédé selon la revendication 1, caractérisé en ce qu'on exécute la ou les lignes (L) d'affaiblissement du papier sur l'une des bandes ou sur les deux bandes destinées à constituer le sachet, sur une installation existante d'ensachage, entre le poste de distribution des bandes et le poste d'introduction du ou des produits à emballer.

-4- Procédé selon la revendication 1, appliqué à des sachets en papier pelable, caractérisé en ce qu'on exécute au moins une ligne (L) d'affaiblissement du papier sur une seule des bandes constituant le sachet.

-5- Procédé selon la revendication 1, appliqué à des sachets en papier totalement enduit, caractérisé en ce qu'on exécute au moins une ligne (L) d'affaiblissement du papier sur chacune des bandes constituant le sachet ; le ou lesdites lignes de chaque bande étant alignées.

-6- Procédé selon la revendication 1, caractérisé en ce qu'on exécute la ou les lignes (L) d'affaiblissement du papier en une zône quelconque de la ou des bandes de papier constituant le sachet (en bordure, au centre, longitudinalement, transversalement...).

-7- Moyens de mise en oeuvre du procédé selon la revendication 1, caractérisés en ce que la ou les lignes (L) d'affaiblissement du papier sont réalisées par passage de la ou des bandes (B1 - B2) constituant le sachet entre une ou des molettes (9) de pression et un ou des galets (10) de contre-appui, disposés sur le parcours de la ou desdites bandes avant leur mise en bobine ou entre la ou les bobines et le poste d'ensachage de l'installation.

-8- Moyens selon la revendication 7, caractérisés en ce que l'un des éléments, molette (9) ou galet (10) est monté réglable en position pour ajuster précisément la profondeur de marquage en fonction du type de papier et de son épaisseur.

-9- Sachet obtenu selon le procédé de la revendication 1, caractérisé en ce qu'il présente au moins une ligne (L) d'affaiblissement du papier sur au moins une des bandes (B1 -B2) constituant le sachet, afin de faciliter son ouverture sans détériorer le contenu et tout en conservant l'étanchéité et la solidité du sachet.

FIG.1

FIG.2

FIG.3

FIG.4

FIG.5

FIG.6

FIG.7

FIG.8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-3 379 364 (PILGER) <br> * Colonne 3, ligne 47 - colonne 4, ligne 65; figures 1,2 * | 1,3,6,7,9 | B 65 B 61/18 <br> B 65 D 75/60 |
| A | FR-A- 783 064 (IVERS-LEE) <br> * Page 1, ligne 64 - page 2, ligne 21; figure 1 * | 1,7,9 | |

**DOMAINES TECHNIQUES RECHERCHES (Int. Cl.5)**

B 65 B
B 65 D

Le présent rapport a été établi pour toutes les revendications

| Lieu de la recherche | Date d'achèvement de la recherche | Examinateur |
|---|---|---|
| LA HAYE | 03-09-1990 | CLAEYS H.C.M. |